# EUROPEAN PATENT APPLICATION

(11) **EP 1 176 216 A2**
(43) Date of publication of application: **30.01.2002**
(21) Application number: 01117844.9
(22) Date of filing: 23.07.2001
(51) Int. Cl.: C12Q 1/68, B09C 1/00

(54) **Nucleic acid, nucleic acid for detecting chlorinated ethylene-decomposing bacteria, probe, method of detecting chlorinated ethylene-decomposing bacteria, and method of decomposing chlorinated ethylene or ethane**

(30) Priority: 24.07.2000 JP 2000227580; 09.03.2001 JP 2001066001
(71) Applicant: Kurita Water Industries Ltd., Tokyo 106-8383 (JP)
(72) Inventor: Nakamura, Kanji, c/oKurita Water Industries Ltd., Tokyo 160-8383 (JP); Ueno, Toshihiro, c/oKurita Water Industries Ltd., Tokyo 160-8383 (JP)
(74) Representative: Goddar, Heinz J., Dr.

(57) **Abstract**

Novel and useful nucleic acids that can be used for detection of chlorinated ethylene-decomposing bacteria and preferentially hybridize to the 16S rRNA or rDNA of chlorinated ethylene-decomposing bacteria, and a method of detection of chlorinated ethylene-decomposing bacteria and a method of decomposition of chlorinated ethylene or ethane using these nucleic acids are disclosed, wherein PCR is performed using nucleic acid comprising 18 ∼ 25 nucleotides that preferentially hybridizes to the 16S rRNA or rDNA of chlorinated ethylene-decomposing bacteria and has any of base sequences No. 1 ∼ 15, a base sequence that has at least 90% homology with any of these base sequences, or a base sequence complementary to any of these base sequences as the primer and the nucleic acid in a sample as the template and the DNA fragment that has been synthesized is detected, and chlorinated ethylene or ethane can be decomposed by introducing the chlorinated ethylene-decomposing bacteria detected by this method to contaminated soil or underground water.

## Description

### Field of the Invention

The present invention relates to nucleic acid that preferentially hybridizes to the 16S rRNA or rDNA of chlorinated ethylene-decomposing bacteria, labeled probe for detecting chlorinated ethylene-decomposing bacteria comprising this nucleic acid, and a method of detecting chlorinated ethylene-decomposing bacteria using this nucleic acid or labeled probe and a method of decomposing chlorinated ethylene or ethane.

### Description of the Related Techniques

As a method of purifying soil or underground water, etc., contaminated by chlorinated ethylene or ethane, there is a method of anaerobic dechlorination of chlorinated ethylene using the chlorinated ethylene-decomposing bacteria that are naturally present in contaminated soil. Moreover, methods of adding these bacteria to contaminated soil or underground water are also known. It is also a known fact that chlorinated ethylene-decomposing bacteria are capable of decomposing not only chlorinated ethylene, but also chlorinated ethane, using the chlorinated ethylene-decomposing enzymes that they possess. However, there are problems with this type of method in that very good treatment results, that is, thorough dechlorination, are not guaranteed.

Therefore, there is a demand for a method of pre-determining whether or not dechlorination can be accomplished when soil or underground water, etc., contaminated by chlorinated ethylene or ethane is to be purified using chlorinated ethylene-decomposing bacteria.

### Summary of the Invention

The object of the present invention is to provide novel and useful nucleic acid that can be used for detection of chlorinated ethylene-decomposing bacteria and that preferentially hybridizes to the 16S rRNA or rDNA of chlorinated ethylene-decomposing bacteria, labeled probe for detection of chlorinated ethylene-decomposing bacteria comprising this nucleic acid, and a method of detecting chlorinated ethylene-decomposing bacteria using this nucleic acid or labeled probe and a method of decomposing chlorinated ethylene or ethane.

The present invention is the following nucleic acid that preferentially hybridizes to the 16S rRNA or rDNA of chlorinated ethylene-decomposing bacteria, labeled probe for detecting chlorinated ethylene-decomposing bacteria comprising this nucleic acid, method of detecting chlorinated ethylene-decomposing bacteria using this nucleic acid or labeled probe, and method of decomposing chlorinated ethylene or ethane:
(1) Nucleic acid comprising 18 ∼ 25 nucleotides, which preferentially hybridizes to the 16S rRNA or rDNA of chlorinated ethylene-decomposing bacteria and has any of base sequence of SEQ ID No. 1 through No. 15, a base sequence having at least 90% homology with these base sequences, or a base sequence complementary to these base sequences.
(2) Nucleic acid comprising 10 ∼ 50 nucleotides that preferentially hybridize to the 16S rRNA or rDNA of chlorinated ethylene-decomposing bacteria wherein the base sequence of at least 10 individual bases in succession is the same as any of base sequences of SEQ ID No. 1 through No. 15 or complementary to these base sequences.
(3) The nucleic acid in above-mentioned (1) or (2), which is used for detection of chlorinated ethylene-decomposing bacteria.
(4) A labeled probe for detection of chlorinated ethylene-decomposing bacteria, comprising nucleic acid in any of above-mentioned (1) through (3) which is labeled by a radioactive element, enzyme, fluorescent substance, antigen, antibody, or chemical substance.
(5) A method of detecting chlorinated ethylene-decomposing bacteria, comprising
   performing PCR (polymerase chain reaction) using the nucleic acid in any of above-mentioned (1) through (3) as the primer and the nucleic acid in a sample as the template and
   detecting the DNA fragment that has been synthesized.
(6) A method of detecting chlorinated ethylene-decomposing bacteria, comprising bringing the labeled probe for detecting chlorinated ethylene-decomposing bacteria in above-mentioned (4) into contact with a sample or nucleic acid prepared from a sample to perform RNA or DNA hybridization, and
   detecting chlorinated ethylene-decomposing bacteria using the label as the indicator.
(7) A method of decomposing chlorinated ethylene or ethane, comprising
   performing the detection of chlorinated ethylene-decomposing bacteria in above-mentioned (5) or (6) using underground water or soil as the sample, and
   introducing the underground water or soil, in which chlorinated ethylene-decomposing bacteria have been detected or cultivation liquid inoculated with these, to soil or underground water contaminated by chlorinated ethylene or ethane.

### Brief Description of the Drawings

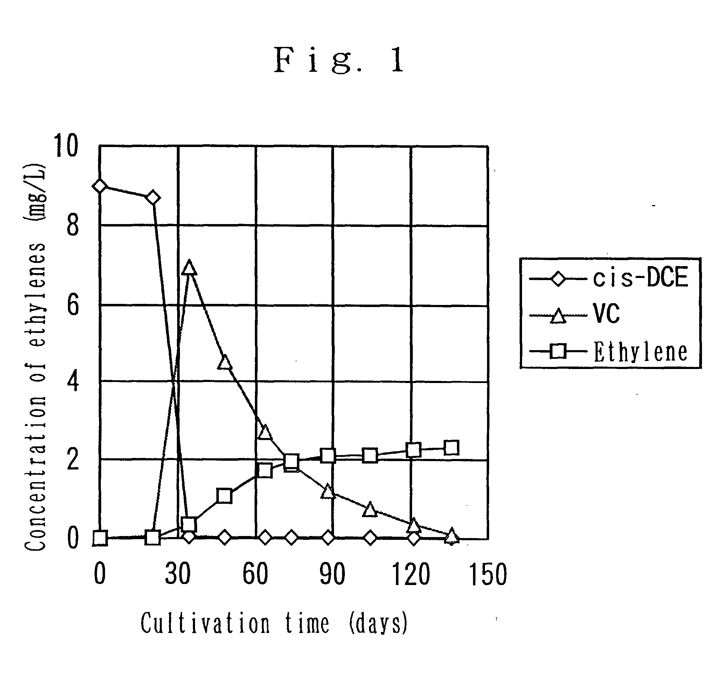

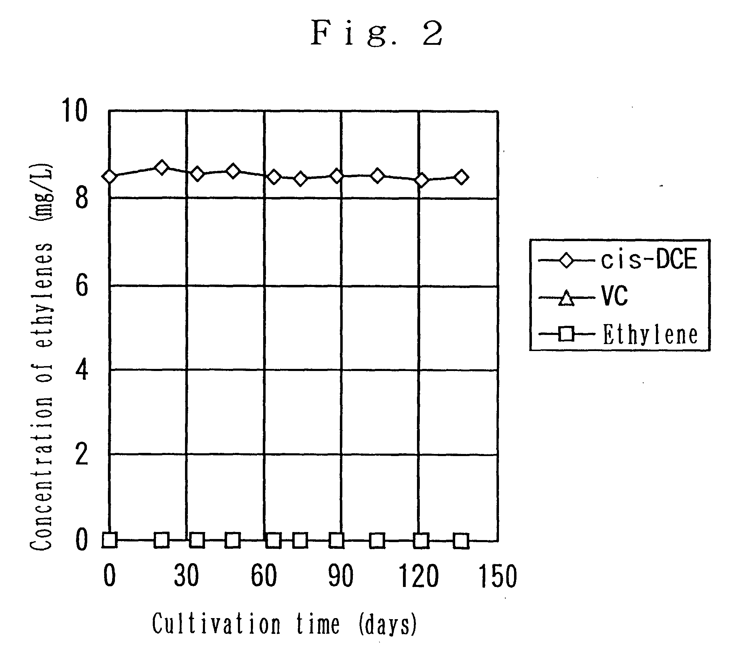

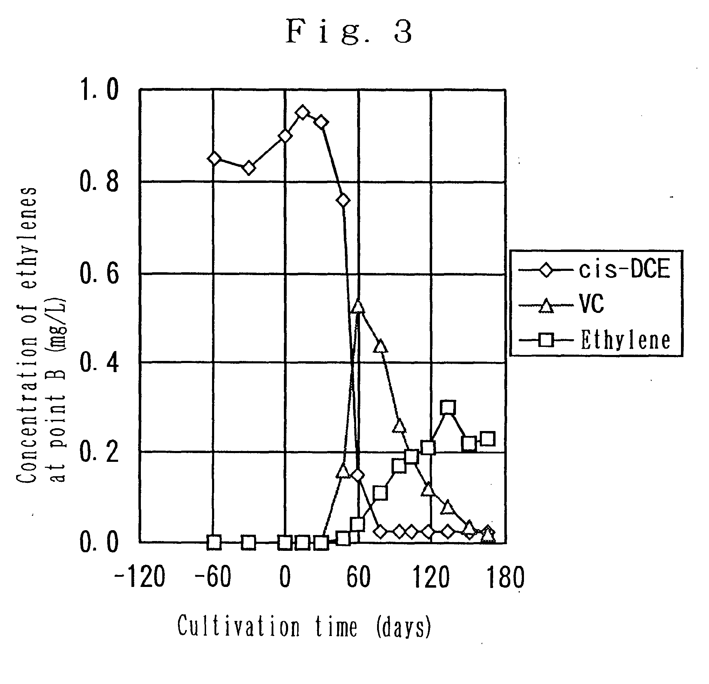

Figure 1 is a graph showing the results of Example 3. Figure 2 is a graph showing the results of the control in Example 3. Figure 3 is a graph showing the results of Example 4.

### Detailed Description of the Disclosure

As a result of studying the reason why treatment results are always not very good with the method of purification of soil or underground water, etc., contaminated by chlorinated ethylene or ethane using chlorinated ethylene-decomposing bacteria, the inventors clarified the fact that treatment results are good when chlorinated ethylene-decomposing bacteria that perform dechlorination live at the treated site, while treatment results cannot be expected when chlorinated ethylene-decomposing bacteria do not live at the treated site. Consequently, it is possible to judge whether or not treatment is thorough by examining the soil and underground water of the subject site and confirming whether or not chlorinated ethylene-decomposing bacteria live at that site.

It is possible to detect chlorinated ethylene-decomposing bacteria and thereby make the above-mentioned judgment by using the nucleic acid of the present invention.

The nucleic acid of the present invention is nucleic acid comprising 18 ∼ 25 nucleotides which preferentially hybridizes to the 16S rRNA or rDNA of chlorinated ethylene-decomposing bacteria and has any of base sequences of SEQ ID No. 1 through No. 15 of the base sequence table, a base sequence having at least 90% homology with these base sequences, or a base sequence complementary to these base sequences.

Moreover, the nucleic acid of the present invention is nucleic acid which comprises 10 ∼ 50 nucleotides, preferably 15 ∼ 35 nucleotides, that preferentially hybridizes to the 16S rRNA or rDNA of chlorinated ethylene-decomposing bacteria and wherein the base sequence of at least 10 individual bases in succession is the same as any of sequences of SEQ ID No. 1 through No. 15 or complementary to these base sequences. An example is nucleic acid having the same base sequence as a base sequence of 10 or more individual bases in succession beginning at any position in base sequence of SEQ ID No. 1 or complementary to these base sequences. A base may be bound upstream and/or downstream of the base sequence that is the same as this base sequence of SEQ ID No. 1 or complementary to these base sequences.

The nucleic acid of the present invention, that is, any of base sequences of SEQ ID No. 1 through No. 15, a base sequence having at least 90% homology with any of these base sequences, a base sequence complimentary to any of these base sequences, or a base sequence wherein the base sequence of at least 10 individual bases in succession is the same as any of base sequence of SEQ ID No. 1 through No. 15 or complementary to these base sequences, can be easily chemically synthesized by conventional methods.

Since the base sequence of the 16S rDNA of chlorinated ethylene-decomposing bacteria has been determined, and then the nucleic acids of the present invention are designed using the specific segment of these sequences, they preferentially hybridize to 16S rRNA or rDNA of chlorinated ethylene-decomposing bacteria.

As specific examples of the above-mentioned chlorinated ethylene-decomposing bacteria, there are bacteria belonging to the genus *Dehalococcoides,* etc.

Specific examples of chlorinated ethylene that can be decomposed or dechlorinated by chlorinated ethylene-decomposing bacteria are tetrachloroethylene, trichloroethylene (TCE), cis-1,2-dichloroethylene, trans-1,2-dichloroethylene, 1,1-dichloroethylene, vinyl chloride, and their dechlorination intermediates, etc. Moreover, specific examples of chlorinated ethanes that can be decomposed or dechlorinated by chlorinated ethylene-decomposing bacteria are 1,2-dichloroethane, monochloroethane, etc.

As will be mentioned later, chlorinated ethylene-decomposing bacteria can be detected easily at specifically high reliability by PCR using the nucleic acids of the present invention as the primer or by hybridization.

The labeled probe for detection of chlorinated ethylene-decomposing bacteria of the present invention is a probe wherein the above-mentioned nucleic acid of the present invention has been labeled with a label, such as a radioactive element, fluorescent substance, chemical substance, antigen, antibody, enzyme, etc. Labels that have usually been used can be used as this label, specific examples being radioactive elements, such as ³²P, etc., phosphors, such as FITC (fluorescence isothiocyanate), rhodamine, etc.; haptenes, such as digoxygenin, etc., enzymes such as alkaline phosphatase, peroxidase, etc., chemical substances, such as biotin, etc. These labels can be introduced to the nucleic acid by conventional methods.

The labeled probe for detecting chlorinated ethylene-decomposing bacteria of the present invention hybridizes with a sample to be checked for the presence of chlorinated ethylene-decomposing bacteria and the chlorinated ethylene-decomposing bacteria that have hybridized with labeled probe can be detected easily with specifically high reliability using this label as the indicator.

The method of detecting chlorinated ethylene-decomposing bacteria of the present invention includes a method of detecting chlorinated ethylene-decomposing bacteria using the above-mentioned nucleic acid of the present invention. That is, PCR is performed using the above-mentioned nucleic acid of the present invention as the primer and the nucleic acid prepared from the sample to be checked for the presence of chlorinated ethylene-decomposing bacteria as the template and if DNA of the expected size is synthesized, it can be concluded that chlorinated ethylene-decomposing bacteria are present in the sample.

PCR can be performed by conventional methods, or it can be performed using a commercial PCR kit. PCR usually uses 2 types of primers, an upper primer and a lower primer. The nucleic acid of the present invention can be used as one or both of the primers. Detection reliability can be improved by performing detection several times using several different types of nucleic acids as the primer.

Moreover, the method of detecting chlorinated ethylene-decomposing bacteria of the present invention includes the method wherein chlorinated ethylene-decomposing bacteria are detected using the above-mentioned labeled probe for detection of chlorinated ethylene-decomposing bacteria of the present invention. That is, it is the method wherein once RNA or DNA hybridization has been performed by bringing the above-mentioned labeled probe for detecting chlorinated ethylene-decomposing bacteria of the present invention into contact with the sample to be checked for presence of chlorinated ethylene-decomposing bacteria or with nucleic acid prepared from this sample, chlorinated ethylene-decomposing bacteria are detected using the label as the indicator. Hybridization can be performed by the same methods as conventional methods.

Detection after hybridization can be performed by conventional methods in accordance with the type of label. For instance, detection can be performed by assaying radioactivity by conventional methods when the probe has been labeled by a radioactive element. Moreover, detection can be performed by measuring the quantity of light by conventional methods when the probe has been labeled by a fluorescent substance. In addition, detection can be performed by assaying enzyme activity by conventional methods when the probe has been labeled by an enzyme. Further, detection can be performed by analyzing chemical substance when the probe has been labeled by chemical substance. Furthermore, detection can be performed by conducting an antigen-antibody reaction using antibody or antigen that reacts specifically with labeled antigen or antibody and determining the reaction product by conventional methods when the probe is labeled by antigen or antibody.

When soil or underground water contaminated by chlorinated ethylene or ethane is to be purified using chlorinated ethylene-decomposing bacteria, it is possible to pre-determine whether or not dechlorination can be throughly accomplished by detecting chlorinated ethylene-decomposing bacteria in the above-mentioned method. Moreover, application of measures including addition of chlorinated ethylene-decomposing bacteria, etc., become possible when chlorinated ethylene-decomposing bacteria have not been detected.

The method of decomposing chlorinated ethylene or ethane of the present invention is the method wherein the above-mentioned detection of ethylene-decomposing bacteria of the present invention is conducted using underground water or soil as the sample, then the underground water or soil, in which chlorinated ethylene-decomposing bacteria have been detected, or cultivation liquid inoculated with these (there are cases hereafter where these are collectively referred to as chlorinated ethylene-decomposing bacteria-detected matter) is introduced to soil or underground water contaminated by chlorinated ethylene or ethane (there are cases hereafter where these are collectively referred to as contaminated environment) and thereby the chlorinated ethylene or ethane is decomposed.

The chlorinated ethylene-decomposing bacteria-detected matter to be introduced to the contaminated environment may be any one which is detected or collected anywhere. For instance, underground water or soil, in which chlorinated ethylene-decomposing bacteria have been detected in a place incontaminated by chlorinated ethylene or ethane, or cultivation liquid inoculated with these, may be introduced to soil or underground water contaminated by chlorinated ethylene or ethane. Moreover, underground water or soil, in which chlorinated ethylene-decomposing bacteria have been detected in a place contaminated by chlorinated ethylene or ethane, or cultivation liquid inoculated with these, may be introduced to a place contaminated by chlorinated ethylene or ethane in the same region or may be introduced to a different place not in the same region.

The method of spreading chlorinated ethylene-decomposing bacteria-detected matter on the surface of contaminated soil, the method of injection into soil from an injection tube or injection well, the method of injection into source of underground water, etc., are given as methods of introducing chlorinated ethylene-decomposing bacteria-detected matter into a contaminated environment. The introduction point may, of course, be the contaminated site, or upstream from the contaminated environment, etc.

When the chlorinated ethylene or ethane is decomposed, there are cases where the underground water or soil, in which chlorinated ethylene-decomposing bacteria have been detected, or cultivation liquid inoculated with these, is simply introduced to the contaminated environment, but depending on the case, water, nutrient source, etc., may also be further introduced. Moreover, if there is not thorough decomposition with 1 introduction, introduction may be repeated. It is also possible to introduce the chlorinated ethylene-decomposing bacteria-detected matter after adding coagulant to coagulate, or after supporting the matter on a carrier.

Thus, a contaminated environment contaminated by chlorinated ethylene or ethane can be purified by decomposing chlorinated ethylene or ethane.

As described above, the nucleic acid of the present invention is novel and useful. The nucleic acids of the present invention have a specific base sequence and hybridizes preferentially to 16S rRNA or rDNA of chlorinated ethylene-decomposing bacteria. Therefore, they can be used for detection of chlorinated ethylene-decomposing bacteria.

The nucleic acids for detection of chlorinated ethylene-decomposing bacteria of the present invention comprise the above-mentioned nucleic acids and therefore, chlorinated ethylene-decomposing bacteria can be easily detected with specifically high reliability by using these nucleic acids.

The labeled probes for detecting chlorinated ethylene-decomposing bacteria of the present invention label the above-mentioned nucleic acid and therefore, it is possible to easily detect with specifically high reliability chlorinated ethylene-decomposing bacteria using this label as the indicator.

The method of detecting chlorinated ethylene-decomposing bacteria of the present invention uses the above-mentioned nucleic acids or labeled probes and therefore, chlorinated ethylene-decomposing bacteria can be detected easily with specifically high reliability.

By means of the method of decomposing chlorinated ethylene or ethane of the present invention, underground water or soil, in which chlorinated ethylene-decomposing bacteria have been detected by the above-mentioned detection method, or cultivation liquid inoculated with these, is introduced to soil or underground water contaminated by chlorinated ethylene or ethane and the chlorinated ethylene or ethane is decomposed. Therefore, chlorinated ethylene or ethane can be easily and efficiently decomposed to purify the environment.

### The Best Mode for Embodying of the Invention

Examples of the present invention will now be described:

### Example 1

Underground water was sampled from a total of 6 places, points A, B and C where conversion to ethylene (dechlorination) is occurring and points D, E and F where conversion to ethylene is not occurring, and DNA was extracted from 100 mL of this underground water as described below:

### (1) Extraction of DNA

After filtering 100 mL underground water with a filter having a pore diameter of 0.2 µm, this filter was introduced to a tube with a capacity of 2 mL. 1 mL zirconia/silica beads (diameter of 0.1 mm) and 1 ml Extraction buffer (100 mM Tris-HCl [pH 8.0], 100 mM sodium EDTA [pH 8.0], 100 mM sodium phosphate [pH 8.0], 1.5 M NaCl) were further added to this tube and treated for 2 minutes with the cell crusher Bead Beater. After repeating freezing and thawing 3 times, 10 µL proteinase K (10 mg/ml) were added and kept at a temperature of 37°C for 30 minutes. Then 250 µL of a 10% SDS solution were added to this liquid and kept at 65°C for 2 hours. The above-mentioned Bead Beater treatment was again performed. This was followed by centrifugation for 10 minutes at 8,000 xg under room temperature. The supernatant was collected. Chloroform extraction of the supernatant was performed. The equivalent amount of isopropanol was added and then it was set aside for 60 minutes at room temperature. Centrifugation was performed for 20 minutes at 8,000 xg under room temperature and the DNA was allowed to precipitate. The precipitate was washed with 70% ethanol and then allowed to dry. Then it was dissolved in 50 µL sterile distilled water.

PCR was performed as described below using this extracted DNA solution and the presence of chlorinated ethylene-decomposing bacteria was examined.

### (2) Amplification of 16S rDNA by PCR

The 16S rDNA was amplified by PCR using 1 µL of the extracted DNA solution obtained by above-mentioned (1) as the template. The total volume of the reaction solution of PCR amplification was brought to 100 µL, and 2.5 U Ex Taq DNA polymerase (Takara Shuzo) and 200 µM dNTP were used. 20 pmol each of 6 sets of primer pairs, where any one of KWI-De1 through KWI-De6 served as the upper primer and Bact1492 (5'-ACGG C/T TACCTTGTTAGGACTT-3') served as the lower primer, and 9 sets of primer pairs, where with Bact0011 (5'-GTTTGATCCTGGCTCAG-3') served as the upper primer and any one of complementary base sequence of KWI-De7 through KWI-De15 served as the lower primer, were used as the primer pair, as shown in Table 1. The rest of the reaction liquid composition was in accordance with the manual accompanying the PCR kit. The PCR reaction was performed by pre-heating at 94°C for 2 minutes, followed by 30 cycles of step 1 at 94°C for 20 seconds, step 2 at 55°C for 30 seconds, and step 3 at 72°C for 2 minutes, and finally, post-extension for 7 minutes at 72°C.

2 µL of the above-mentioned PCR reaction liquid were submitted to agarose electrophoresis and it was concluded that chlorinated ethylene-decomposing bacteria were present if DNA fragment of the expected size was synthesized. The results are shown in Table 1.

**Table 1.**

| Results of detecting chlorinated ethylene-decomposing bacteria from underground water | | | | | | | | | |
|---|---|---|---|---|---|---|---|---|---|
| No | Upper Primer | Lower Primer | Length of synthetic | Point | | | | | |
| | | | DNA (kb) | A | B | C | D | E | F |
| 1 | KWI-De1 | Bact1492 | 1.38 | ○ | × | ○ | × | × | × |
| 2 | KWI-De2 | Bact1492 | 1.34 | ○ | ○ | ○ | × | × | × |
| 3 | KWI-De3 | Bact1492 | 1.31 | ○ | ○ | ○ | × | × | × |
| 4 | KWI-De4 | Bact1492 | 1.28 | ○ | ○ | × | × | × | × |
| 5 | KWI-De5 | Bact1492 | 1.26 | ○ | ○ | ○ | × | × | × |
| 6 | KWI-De6 | Bact1492 | 1.24 | ○ | ○ | ○ | × | × | × |

| | | **Base sequence complementary to** | | | | | | | |
|---|---|---|---|---|---|---|---|---|---|
| 7 | Bact0011 | KWI-De7 | 0.91 | × | ○ | ○ | × | × | × |
| 8 | Bact0011 | KWI-De8 | 0.82 | ○ | ○ | ○ | × | × | × |
| 9 | Bact0011 | KWI-De9 | 0.80 | ○ | ○ | ○ | × | × | × |
| 10 | Bact0011 | KWI-De10 | 0.98 | ○ | × | ○ | × | × | × |
| 11 | Bact0011 | KWI-De11 | 1.01 | ○ | ○ | ○ | × | × | × |
| 12 | Bact0011 | KWI-De12 | 1.10 | ○ | ○ | ○ | × | × | × |
| 13 | Bact0011 | KWI-Del3 | 1.22 | ○ | ○ | ○ | × | × | × |
| 14 | Bact0011 | KWI-De14 | 1.24 | ○ | ○ | × | × | × | × |
| 15 | Bact0011 | KWI-De15 | 1.40 | ○ | ○ | ○ | × | × | × |

| | | | | | | | | | |
|---|---|---|---|---|---|---|---|---|---|
| ○ : Synthesis of DNA observed | | | | | | | | | |
| × : Synthesis of DNA not observed | | | | | | | | | |

The base sequence of KWI-De1 ∼ KWI-De15 in Table 1 are as shown in Table 2.

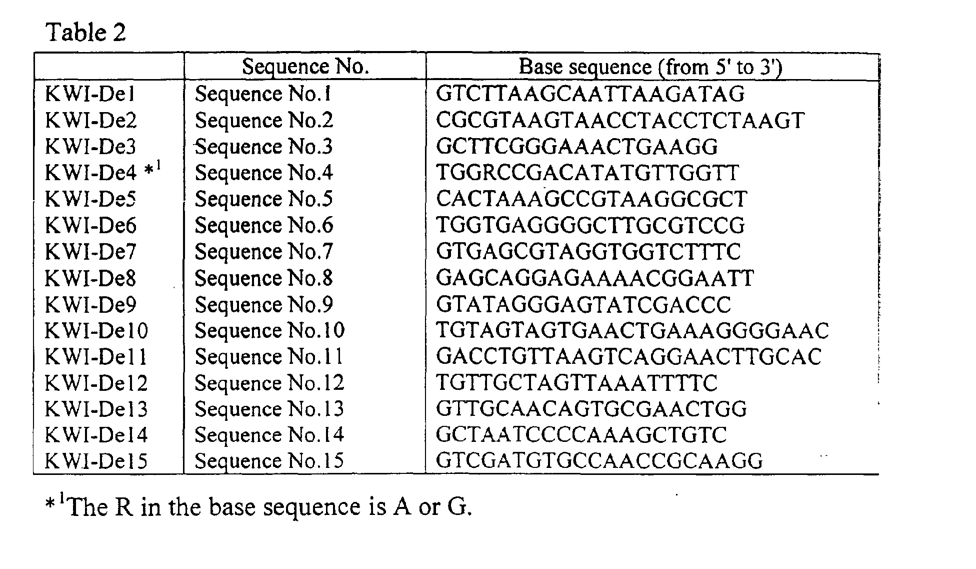

Based on the results in Table 1, DNA synthesis was observed in all but 5 of 45 times by the above-mentioned PCR and electrophoresis at points A, B and C, where ethylene conversion is occurring. On the other hand, no DNA synthesis whatsoever was observed at points D, E and F, where no ethylene conversion whatsoever is occurring. Based on these results, chlorinated ethylene-decomposing bacteria are always present and they can be monitored at points where ethylene conversion is occurring.

### Example 2

### (1) Detection by Light Cycler

PCR detection of even higher reliability was conducted on the extracted DNA solutions of Example 1 using the Light Cycler made by Roche Diagnostics Co., Ltd.

In this case, KWI-De8 was used as the upper primer and oligonucleotide complementary to KWI-De15 was used as the lower primer. Moreover, KWI-De10 labeled with FITC (fluorescence isothiocyanate) at the 3' terminal and KWI-De11 phosphorylated at the 3' terminal and labeled with FITC at the 5' terminal were used as the hybridization probe. The PCR reaction was carried out using Light Cycler DNA Master Hybridization Probes Kit (Trademark) in accordance with the manual thereof. The reaction conditions are shown in Tables 3 ∼ 6.

**Table 3.**

| Denaturation | | | | |
|---|---|---|---|---|
| Number of cycles = 1 | | | | |
| Segment | Target temperature (°C) | Storage time (s) | Speed of temp. change (°C/s) | Fluorescence detected |
| 1 | 95 | 120 | 20 | None |

**Table 4.**

| Denaturation | | | | |
|---|---|---|---|---|
| Number of cycles = 50 (segment 1 → 2 → 3 → back to 1) | | | | |
| Segment | Target temperature (°C) | Storage time (s) | Speed of temp. change (°C/s) | Fluorescence detected |
| 1 | 95 | 0 | 20 | None |
| 2 | 54 | 15 | 20 | Detected once |
| 3 | 72 | 30 | 2 | None |

**Table 5.**

| Denaturation | | | | |
|---|---|---|---|---|
| Number of cycles = 1 | | | | |
| Segment | Target temperature (°C) | Storage time (s) | Speed of temp. change (°C/s) | Fluorescence detected |
| 1 | 95 | 0 | 20 | None |
| 2 | 44 | 10 | 20 | None |
| 3 | 85 | 0 | 0.2 | Continuously detected |

**Table 6.**

| Denaturation | | | | |
|---|---|---|---|---|
| Number of cycles = 1 | | | | |
| Segment | Target temperature (°C) | Storage time (s) | Speed of temp. change (°C/s) | Fluorescence detected |
| 1 | 40 | 30 | 20 | None |

The results showed that the desired DNA can be synthesized by PCR and therefore, chlorinated ethylene-decomposing bacteria are present in the underground water at points A, B and C. Nevertheless, it was concluded that chlorinated ethylene-decomposing bacteria are not present at points D, E and F because the desired DNA was not synthesized. Thus, it is clear that the primers in Table 2 can be used as the hybridization probe as well.

### Example 3

100 g soil and 50 mL underground water contaminated by cis-dichloroethylene (cis-DCE) were introduced to a vial with a capacity of 150 mL. Lactic acid was added to a concentration of 100 mg/L and then [the bottle] was closed with a butyl rubber stopper and sealed with an aluminum cap. Two of these same vials were used. Bacterium suspension in which chlorinated ethylene-decomposing bacterium genes had been detected were transferred in one vial to a final concentration of chlorinated ethylene-decomposing [bacterium] genes of 10⁵ copies/mL. Moreover, [genes] were not transferred to the other vial, which served as the control. These vials were set aside at 30°C for cultivation. They were periodically sampled and the concentration of ethylenes in the vials were determined. The results are shown in Figures 1 and 2.

There was marked chlorinated ethylene decomposition and vinyl chloride (VC) was first detected approximately 20 days after starting the experiment when bacterium suspension in which chlorinated ethylene-decomposing [bacterium] genes had been detected was added (Figure 1). Thereafter, the VC was also decomposed, with there being complete conversion to ethylene in approximately 135 days. On the other hand, no vinyl chloride or ethylene whatsoever was detected throughout the experimental period in the case of the control (Figure 2).

Based on the above-mentioned results, it is clear that adding a liquid in which chlorinated ethylene-decomposing bacteria have been detected has the effect of promoting the chlorinated ethylene-decomposition reaction.

### Example 4

Wells (A and B) were placed in 2 places that were 1 m apart in an area contaminated by chlorinated ethylene. Water was pumped from point B at 3 L/min and this water was introduced to point A. When introduced to point A, lactic acid was added at a concentration of 100 mg/L. The contaminated aquifer was 3 m below the ground and the aquifer was 4 m thick.

The underground water was periodically sampled at point B and the concentration of ethylenes was determined. The results are shown in Figure 3. The axis of abscissas shows the time that had lapsed and the 0 point is the time when 50 L of liquid in which chlorinated ethylene-decomposing bacteria had been detected (gene concentration: 10⁷ copies/mL) had been introduced from point A.

As is clear from the results in Figure 3, no decomposition of dichloroethylene whatsoever was seen prior to introduction, but there was marked decomposition 20 days after introduction, with conversion to ethylene being 100% after approximately 170 days.

Based on the above-mentioned results, it is clear that adding liquid in which chlorinated ethylene-decomposing bacteria have been detected has the effect of promoting the chlorinated ethylene decomposition reaction, even in areas contaminated by chlorinated ethylene.

### Sequence Listing

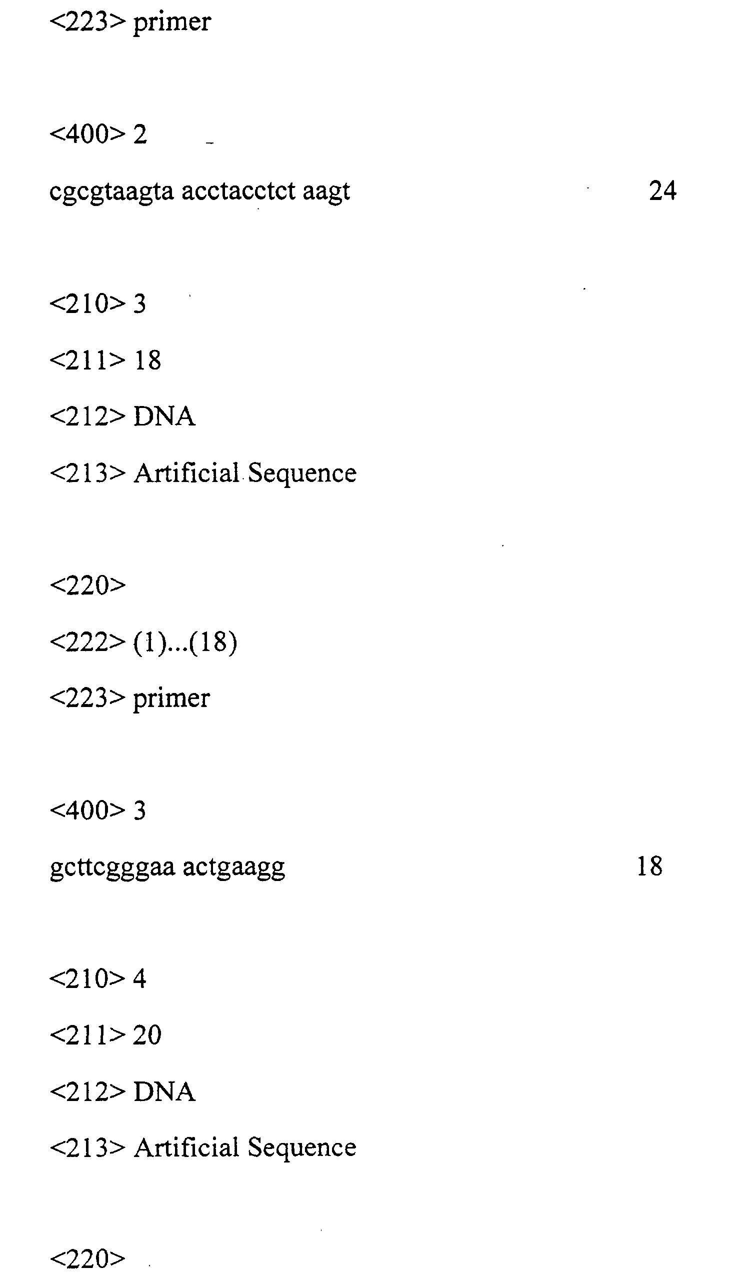

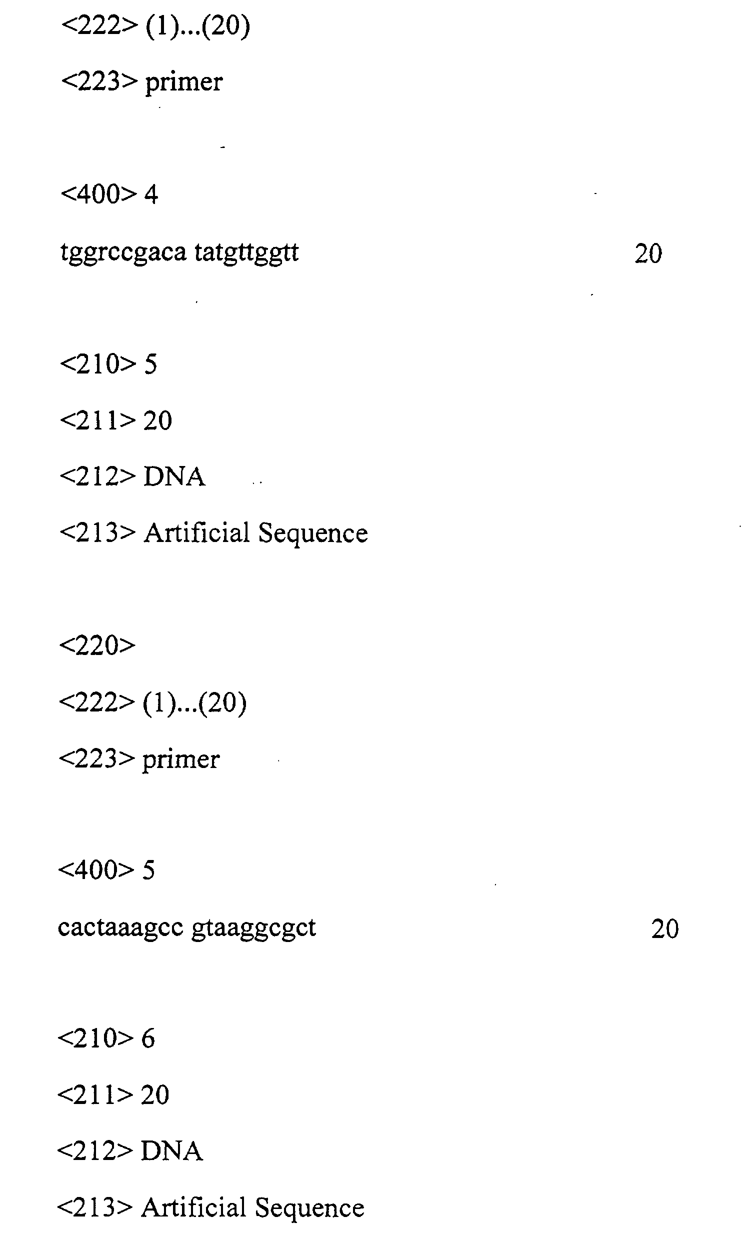

<110> KURITA WATER INDUSTRIES LTD.

<120> Nucleic Acid, Nucleic Acid for Detecting Chlorinated Ethylene-
Decomposing Bacteria, Probe, Method of Detecting Chlorinated Ethylene-
Decomposing Bacteria, and Method of Decomposing Chlorinated Ethylene
or Ethane

<130> KWI00340

<150> JP 2000-227580
<151> 2000-07-24

<151> 2001-03-09

<160> 17

<210> 1
<211> 20
<212> DNA
<213> Artificial Sequence

<220>
<222> (1)...(20)
<223> primer

<400> 1
gtcttaagca attaagatag                                                    20

<210> 2
<211> 24
<212> DNA
<213> Artificial Sequence

<220>
<222> (1)...(24)

<220>
<222> (1)...(20)
<223> primer

<400> 6
tggtgagggg cttgcgtccg 20

<210> 7
<211> 20
<212> DNA
<213> Artificial Sequence

<220>
<222> (1)...(20)
<223> primer

<400> 7
gtgagcgtag gtggtctttc 20

<210> 8
<211> 20
<212> DNA
<213> Artificial Sequence

<220>
<222> (1)...(20)
<223> primer

<400> 8
cagcaggaga aaacggaatt 20

<210> 9
<211> 19
<212> DNA
<213> Artificial Sequence

<220>
<222> (1)...(19)
<223> primer

<400> 9
gtatagggag tatcgaccc 19

<210> 10
<211> 25
<212> DNA

<213> Artificial Sequence

<220>
<222> (1)...(25)
<223> primer

<400> 10
tgtagtagtg aactgaaagg ggaac 25

<210> 11
<211> 25
<212> DNA
<213> Artificial Sequence

<220>
<222> (1)...(25)
<223> primer

<400> 11
gacctgttaa gtcaggaact tgcac 25

<210> 12
<211> 19

<212> DNA
<213> Artificial Sequence

<220>
<222> (1)...(19)
<223> primer
<400> 12
tgttgctagt taaattttc 19

<210> 13
<211> 20
<212> DNA
<213> Artificial Sequence

<220>
<222> (1)...(20)
<223> primer

<400> 13
gttgcaacag tgcgaactgg 20

<210> 14
<211> 19

<212> DNA
<213> Artificial Sequence

<220>
<222> (1)...(19)
<223> primer

<400> 14
gctaatcccc aaagctgtc 19

<210> 15
<211> 21
<212> DNA
<213> Artificial Sequence

<220>
<222> (1)...(21)
<223> primer

<400> 15
gtcgatgtgc caaccgcaag g 21

<210> 16

<211> 21

<212> DNA

<213> Artificial Sequence

<220>

<222> (1)...(21)

<223> primer

<400> 16

acggytacct tgttaggact t 21

<210> 17

<211> 17

<212> DNA

<213> Artificial Sequence

<220>

<222> (1)...(17)

<223> primer

<400> 17

gtttgatcct ggctcag 17

The features disclosed in the foregoing description, in the claims and/or in the accompanying drawings may, both separately and in any combination thereof, be material for realising the invention in diverse forms thereof.

## Claims

1. Nucleic acid comprising 18 ∼ 25 nucleotides, which preferentially hybridizes to the 16S rRNA or rDNA of chlorinated ethylene-decomposing bacteria and has any of base sequences of SEQ ID No. 1 through No. 15, a base sequence having at least 90% homology with these base sequences, or a base sequence complementary to these base sequences.

2. Nucleic acid comprising 10 ∼ 50 nucleotides that preferentially hybridizes to the 16S rRNA or rDNA of chlorinated ethylene-decomposing bacteria, wherein the base sequence of at least 10 individual bases in succession is the same as any of base sequences of SEQ ID No. 1 through No. 15 or complementary to these base sequences.

3. The nucleic acid in claim 1 or 2, which is used for detection of chlorinated ethylene-decomposing bacteria.

4. A labeled probe for detection of chlorinated ethylene-decomposing bacteria, comprising nucleic acid in any of claims 1 through 3 which is labeled by a radioactive element, enzyme, fluorescent substance, antigen, antibody, or chemical substance.

5. A method of detecting chlorinated ethylene-decomposing bacteria, comprising
performing PCR (polymerase chain reaction) using the nucleic acid in any of claims 1 through 3 as the primer and the nucleic acid in a sample as the template and detecting the DNA fragment that has been synthesized.

6. A method of detecting chlorinated ethylene-decomposing bacteria, comprising
bringing the labeled probe for detecting chlorinated ethylene-decomposing bacteria in claim 4 into contact with a sample or nucleic acid prepared from a sample to perform RNA or DNA hybridization, and
detecting chlorinated ethylene-decomposing bacteria using the label as the indicator.

7. A method of decomposing chlorinated ethylene or ethane, comprising
performing the detection of chlorinated ethylene-decomposing bacteria in claim 5 or 6 using underground water or soil as the sample, and
introducing the underground water or soil, in which chlorinated ethylene-decomposing bacteria have been detected, or cultivation liquid inoculated with these, to soil or underground water contaminated by chlorinated ethylene or ethane.
